# EUROPEAN PATENT SPECIFICATION

(11) **EP 3 788 994 B1**
(45) Date of publication and mention of the grant of the patent: **23.08.2023**
(21) Application number: 19796695.5
(22) Date of filing: 24.04.2019
(51) Int. Cl.: A61F 9/007, A61B 90/50, A61B 34/30, A61B 1/00, A61B 90/00, A61B 17/00

(54) **INTRAOCULAR SURGERY INSTRUMENT HOLDER**
HALTER FÜR EIN INSTRUMENT FÜR DIE INTRAOKULARE CHIRURGIE
PORTE-INSTRUMENT DE CHIRURGIE INTRAOCULAIRE

(30) Priority: 02.05.2018 JP 2018088603
(43) Date of publication of application: 10.03.2021
(73) Proprietor: Riverfield Inc., Minato-ku Tokyo 1070052 (JP)
(72) Inventor: TADANO, Kotaro, Tokyo 152-8550 (JP); KIMURA, Shintaro, Tokyo 152-8550 (JP); YAMAUCHI Gai, Tokyo 152-8550 (JP); SONODA Koh-hei, Fukuoka-shi, Fukuoka 819-0395 (JP); NAKAO Shintaro, Fukuoka-shi, Fukuoka 819-0395 (JP)
(74) Representative: Banse & Steglich Patentanwälte PartmbB
(86) International application number: PCT/JP2019/017322
(87) International publication number: WO 2019/212018

(56) References cited:
- WO-A1-2015/064425
- WO-A1-2017/122202
- WO-A1-2018/050207
- WO-A1-2018/051665
- WO-A1-2018/052796
- JP-A- 2007 307 122
- JP-A- 2007 307 122
- JP-A- 2009 136 538
- US-A- 5 410 638
- US-A1- 2014 316 436
- US-A1- 2015 073 597
- US-A1- 2015 157 497
- US-B2- 8 004 229

## Description

### Technical Field

The present invention relates to an intraocular surgery instrument holder.

### Background Art

In recent years, endoscopic surgery has widely been used in a surgical operation such as laparoscopic surgery. In such surgery, it is difficult for a surgeon to hold an endoscope with one hand and also apply a treatment by operating a surgical instrument with the other hand. Further, even in a case where an assistant holds the endoscope, the surgeon is given considerable stress due to an operation of the endoscope not following intentions of the surgeon, much hands movement, or the like. Thus, an endoscope holder has been developed which holds the endoscope as a third hand of the surgeon. Non Patent Literature 1 discloses an endoscope holder including a link mechanism, a driving unit driving the link mechanism, and a hand controller and a foot controller as operation units by which a surgeon performs operations, in which whether the endoscope is moved automatically or manually is switched by a button.

Document JP 2007 307122 A discloses an ophthalmologic surgical operation supporting apparatus for assisting an operation of a surgical instrument by an operator for aligning a distal end of a surgical instrument to be inserted into an eye through a puncture opened in a patient's eyeball. Furthermore, an xyz-fine adjustment unit is disclosed which corresponds to the translational driving unit in the present invention, and an xyz-rough adjustment unit. A brake mechanism in the xyz-fine adjustment unit is provided which is used to inhibit (i.e., restrain) the movement of the xyz-fine adjustment unit.

Document WO 2018/052796 A1 discloses a surgical system comprising a base movable relative to a floor surface; a remotely controllable arm extending from the base and configured to support a surgical tool, the arm having a powered joint operable to move the surgical tool when the surgical tool is supported by the remotely controllable arm, a positioning indicator; and a processor communicatively coupled to the positioning indicator and the remotely controllable arm. The processor is configured to operate the positioning indicator to direct a manual repositioning of the base relative to the floor surface; and to operate the powered joint to maintain a position of a distal portion of the remotely controllable arm during the manual repositioning.

### Citation List

### Non Patent Literature

Non Patent Literature 1: J. M. Sackier and one other, "Robotically assisted laparoscopic surgery", Surgical Endoscopy January, USA, 1994, Volume 8, Issue 1, pp.63-66.

### Summary of Invention

### Technical Problem

Recently, in ophthalmic surgery such as vitreoretinal surgery, a surgical instrument holder (intraocular surgery instrument holder) has been developed, the surgical instrument holder holding, as a third hand of a surgeon, a surgical instrument such as an endoscope. Safety has to be particularly taken into consideration in an intraocular surgery instrument holder. That is, it is necessary to lower risks of damaging an eyeball by a surgical instrument due to malfunction as much as possible.

As described above, in an endoscope holder disclosed in Non Patent Literature 1, the endoscope may be moved automatically or manually. In general, the endoscope is manually moved (coarse movement mode) in a case of positioning the endoscope or of largely changing the field of view, and the endoscope is automatically moved (fine movement mode) in a case where the field of view of the endoscope is finely moved in giving a treatment. However, because the endoscope holder disclosed in Non Patent Literature 1 is a structure for switching whether the same link mechanism is moved manually or automatically, the physical movable range is the same in the coarse movement mode and the fine movement mode. Thus, when the endoscope holder disclosed in Non Patent Literature 1 is used as the intraocular surgery instrument holder, it is possible that the surgical instrument largely moves and damages an eyeball if by any case malfunction occurs during an operation in the fine movement motion, and there has been room for improvement.

This application has been made in consideration of the above background, and an object is to provide an intraocular surgery instrument holder that may lower risks of damaging an eyeball by a surgical instrument due to malfunction as much as possible.

### Solution to Problem

An intraocular surgery instrument holder according to the present invention as claimed in claim 1 includes: a translational driving unit that is a driving mechanism configured to be capable of movement with multiple degrees of freedom; an arm unit that is a passive motion mechanism coupled with the translational driving unit and is configured to be capable of movement with multiple degrees of freedom; and a surgical instrument holding unit that is a passive gimbal mechanism coupled with one end of the arm unit, and is configured to hold an intraocular surgery instrument, and to be capable of movement with two degrees of rotational freedom with an opening part of a hole being a fixed point, the hole being formed in an eyeball for insertion of the intraocular surgery instrument, in which a brake mechanism for inhibiting movement of the arm unit is provided to the arm unit, wherein when the translational driving unit is driven, the brake mechanism is actuated inter-connectedly. Further embodiments are defined by the dependent claims. Methods are not claimed.

In the intraocular surgery instrument holder according to one embodiment of the present invention, the translational driving unit as the driving mechanism with multiple degrees of freedom may be used as a movement mechanism for fine movement, and the arm unit as the passive motion mechanism with multiple degrees of freedom may be used as a movement mechanism for coarse movement. As described above, the movement mechanism for fine movement and the movement mechanism for coarse movement are divided from each other as separate mechanisms, and physical movable ranges may thereby separately be set for fine movement and coarse movement. By setting a movable range of the translational driving unit short compared to a movable range of the arm unit, even if the translational driving unit malfunctions when the translational driving unit causes the intraocular surgery instrument to automatically perform fine movement, the eyeball is not damaged due to large movement of the intraocular surgery instrument. Further, because the arm unit is provided with the brake mechanism, movement of the arm unit may be restricted in a case where the arm unit is not desired to be moved such as a case where the translational driving unit causes an endoscope to perform fine movement.

A configuration is made such that the brake mechanism is actuated inter-connectedly when the translational driving unit is driven, and movement of the arm unit may more certainly be inhibited in fine movement.

In the arm unit as the passive motion mechanism with multiple degrees of freedom, "multiple degrees of freedom" means at least three degrees of freedom. As described above, the arm unit is configured as the passive motion mechanism with at least three degrees of freedom, and movement of the intraocular surgery instrument may thereby smoothly be performed in coarse movement.

A self-weight compensation mechanism for supporting self-weights of the arm unit, the surgical instrument holding unit, and the intraocular surgery instrument is provided to the arm unit, and a force of the self-weight compensation mechanism is exerted to lift a tip end of the arm unit perpendicularly upward with respect to the arm unit in a state where the brake mechanism is not actuated. When actuation of the brake mechanism is canceled in a state where a surgeon does not support the arm unit by his/her hand, the tip end of the arm unit is lifted perpendicularly upward due to exertion of the force of the self-weight compensation mechanism. Accordingly, in an emergency such as a case where a patient brings his/her head up in a state where the intraocular surgery instrument is inserted into an interior of the eyeball, the intraocular surgery instrument may quickly be removed from the interior of the eyeball.

### Advantageous Effects of Invention

The present invention may lower risks of damaging an eyeball by an intraocular surgery instrument due to malfunction as much as possible.

### Brief Description of the Drawings

Fig. 1 is a schematic diagram illustrating a configuration of an intraocular surgery instrument holder according to this embodiment.
Fig. 2 is a block diagram illustrating a control mechanism of the intraocular surgery instrument holder according to this embodiment.
Fig. 3 is a schematic diagram explaining movement of field of view of an endoscope in up, down, left, and right directions in a fine movement mode.
Fig. 4 is a schematic diagram explaining enlargement and reduction (zoom-in and zoom-out) of the field of view of the endoscope in the fine movement mode.

### Description of Embodiments

In the following, the present invention will be described through an embodiment of the invention; however, the invention according to Claims is not limited to the following embodiment. Further, not all configurations described in the embodiment are necessarily required as means for solving problems. For clarification of descriptions, the following descriptions and drawings are appropriately omitted and simplified. In each of the drawings, the same reference characters are given to the same elements, and repeated descriptions will be omitted as needed.

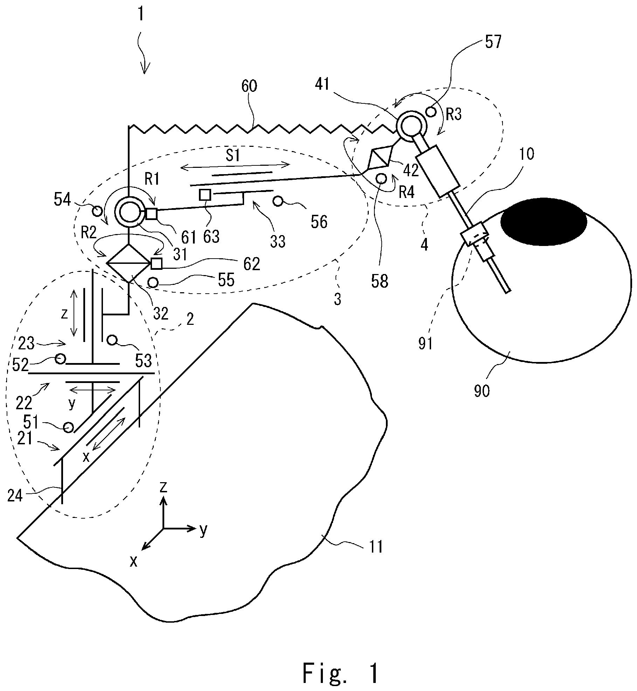

Fig. 1 is a schematic diagram illustrating a configuration of an intraocular surgery instrument holder according to this embodiment. As illustrated in Fig. 1, an intraocular surgery instrument holder 1 includes a translational driving unit 2, an arm unit 3, and a surgical instrument holding unit 4.

The translational driving unit 2 is a driving mechanism configured to be capable of movement with multiple degrees of freedom. That is, the translational driving unit 2 is a mechanism for actively performing, by a driving source, movement of field of view of an endoscope 10 as an intraocular surgery instrument in giving a treatment. As the translational driving unit 2, an xyz stage with three degrees of translational freedom may be used. The xyz stage is configured by combining three automatic linear motion stages (z stage 21, y stage 22, and z stage 23) respectively arranged in directions of x axis, y axis, and z axis, for example. Each of the x stage 21, y stage 22, and z stage 23 has a guide rail and a movement unit moving along the guide rail. The movement unit of each of the x stage 21, y stage 22, and z stage 23 is driven by a highly precise motor such as a stepping motor or a servomotor, for example. Note that a rotational motion of the motor is converted into a rectilinear motion (linear motion) by a ball screw, for example. A movable range of the translational driving unit 2 is to an extent that is necessary and sufficient for observation of an interior of an eyeball 90 by the endoscope 10, for example.

To the translational driving unit 2, displacement sensors 51, 52, and 53 are attached which are for respectively detecting displacement amounts (distance displacements) in x-axis direction, y-axis direction, and z-axis direction. As the displacement sensors 51, 52, and 53, for example, an incremental optical encoder may be used.

The translational driving unit 2 has a fixing mechanism 24 for fixing the intraocular surgery instrument holder 1 to a side periphery of a surgical table 11. That is, the fixing mechanism 24 is installed in the guide rail of the x stage 21 in the translational driving unit 2. Note that the fixing mechanism 24 may be anything as long as it may stably fix the intraocular surgery instrument holder 1 to the surgical table 11.

In the translational driving unit 2, the arm unit 3 is coupled with an end portion on the opposite side to an end portion fixed to the surgical table 11. The arm unit 3 is a passive motion mechanism configured to be capable of movement with multiple degrees of freedom. That is, the arm unit 3 is a mechanism for passively performing movement of the position of the endoscope 10 by a force of a hand of a surgeon when positioning (setting) of the endoscope 10 is performed or when the field of view of the endoscope 10 has to be largely changed in giving a treatment. Note that in this embodiment, the arm unit 3 is a passive motion mechanism configured to be capable of moving with two degrees of rotational freedom and one degree of translational freedom in a polar coordinate system. The arm unit 3 has two rotational joints 31 and 32 and one linear motion joint 33. The rotational joint 31 rotates the arm unit 3 in a pitch direction (arrow R1). The rotational joint 32 rotates the arm unit 3 in a yaw direction (arrow R2). The linear motion joint 33 is configured to be capable of changing the length by extending and contracting the arm unit 3 by translation in the direction of an arrow S1. Each of the joints in the arm unit 3 is a passive motion joint (a joint not driven) so as to be capable of being passively moved. The movable range of the arm unit 3 is set wider than the movable range of the translational driving unit 2.

To the arm unit 3, displacement sensors 54 and 55 and a displacement sensor 56 are attached, the displacement sensors 54 and 55 being for respectively detecting displacement amounts (angular displacements) of the rotational joints 31 and 32, the displacement sensor 56 being for detecting a displacement amount (distance displacement) of the linear motion joint 33. As the displacement sensors 54 and 55, for example, an absolute optical encoder may be used. Further, as the displacement sensor 56, for example, an incremental optical encoder may be used.

Brake mechanisms 61, 62, and 63 inhibiting motions of the joints are respectively provided to the rotational joints 31 and 32 and the linear motion joint 33 in the arm unit 3. As the brake mechanisms 61 and 62 for the rotational joints 31 and 32, electromagnetic brakes causing a braking effect by an electromagnetic force may be used. As the brake mechanism 63 for the linear motion joint 33, an air-pressure brake causing a braking effect by an air pressure may be used. For example, when movement of the position of the endoscope 10 as the intraocular surgery instrument is performed by the translational driving unit 2 in giving a treatment, brakes for the respective joints (the rotational joints 31 and 32 and the linear motion joint 33) are actuated by the brake mechanisms 61, 62, and 63, and passive movement of the arm unit 3 is thereby inhibited.

A self-weight compensation mechanism 60 is provided to the arm unit 3. The self-weight compensation mechanism 60 is a mechanism for supporting, by an elastic force of a spring, the self-weights of the arm unit 3, the surgical instrument holding unit 4, and the endoscope 10 as the intraocular surgery instrument. The elastic force of the spring in the self-weight compensation mechanism 60 may be set to be balanced with the self-weights of the arm unit 3, the surgical instrument holding unit 4, and the endoscope 10 as the intraocular surgery instrument but may be set greater than the self-weights. In a case where the elastic force of the spring in the self-weight compensation mechanism 60 is set greater than the self-weights, the force of the self-weight compensation mechanism 60 is exerted to lift a tip end of the arm unit 3 perpendicularly upward with respect to the arm unit 3 in a state where the brake mechanisms are not actuated. Note that in a case where the elastic force of the spring in the self-weight compensation mechanism 60 is set greater than the self-weights, the elastic force of the spring is to an extent that the surgeon lightly supports the arm unit 3 by his/her hand and the arm unit 3 may thereby be made to not lift up.

In a case where the elastic force of the spring in the self-weight compensation mechanism 60 is set greater than the self-weights as described above, the self-weight compensation mechanism 60 may be caused to function as a safety device in an emergency. In this case, in a state where the brakes are not actuated for the respective joints (the rotational joints 31 and 32 and the linear motion joint 33) of the arm unit 3, the tip end of the arm unit 3 is pulled upward by the elastic force of the spring, the rotational joint 31 rotates in the pitch direction, and the tip end of the arm unit 3 is lifted up. That is, when actuation of the brake mechanisms is canceled in a state where the surgeon does not support the arm unit by his-her hand, the tip end of the arm unit is lifted perpendicularly upward due to exertion of the force of the self-weight compensation mechanism. Accordingly, in an emergency such as a case where a patient brings his/her head up in a state where the endoscope is inserted into the interior of the eyeball, the endoscope may quickly be removed from the interior of the eyeball, and the eyeball 90 may thus not be damaged.

The surgical instrument holding unit 4 is coupled with one end (a part around the tip end) of the arm unit 3. The surgical instrument holding unit 4 is for holding the endoscope 10 as the intraocular surgery instrument. Further, the surgical instrument holding unit 4 is a passive gimbal mechanism configured to be capable of movement with two degrees of rotational freedom with an opening part 91 of a hole being a fixed point (pivot point), the hole being formed in the eyeball 90 for insertion of the endoscope 10 as the intraocular surgery instrument. That is, when receiving a translation motion from the translational driving unit 2 or the arm unit 3, the surgical instrument holding unit 4 functions to convert the translation motion into a self-rotation motion, around the fixed point as the center, of the endoscope 10 as the intraocular surgery instrument, the endoscope 10 being inserted into the interior of the eyeball 90, and has rotational joints 41 and 42.

The rotational joint 41 rotates the surgical instrument holding unit 4 in a pitch direction (arrow R3). The rotational joint 42 rotates the surgical instrument holding unit 4 in a yaw direction (arrow R4). Each of the joints (rotational joints 41 and 42) in the surgical instrument holding unit 4 is a passive motion joint so as to be capable of moving while following movement of the translational driving unit 2 or the arm unit 3. The rotational joints 41 and 42 are made as the passive motion joints, and the posture of the endoscope 10 as the intraocular surgery instrument, the endoscope 10 being inserted into the eyeball 90, thereby flexibly changes in response to the motion of the eyeball 90. Accordingly, a load on the eyeball 90 of the patient may be decreased.

To the surgical instrument holding unit 4, displacement sensors 57 and 58 are attached which are for respectively detecting displacement amounts (angular displacements) of the rotational joints 41 and 42. As the displacement sensors 57 and 58, for example, an absolute optical encoder may be used.

Note that the endoscope 10 as the intraocular surgery instrument, the endoscope 10 being held by the intraocular surgery instrument holder 1 according to this embodiment, is a common endoscope used for observation of the interior of the eyeball 90. For example, the endoscope 10 is configured to include a flexible insertion unit having an image-capturing unit in a tip end portion to be inserted into an eye, an operation unit performing control of an optical system, and a connection unit connected with the operation unit and connecting a light source or the like with the operation unit. The image-capturing unit is configured to include an optical unit formed with an object lens and so forth, a solid state image sensor, and a zoom mechanism unit including an actuator controlling a lens of the optical unit to enlarge or reduce an image obtained by the image-capturing unit. A light guide is provided adjacently to the object lens in the tip end portion of the insertion unit. The light guide illuminates an inside of a body by light guided from the above-described light source.

Next, a control mechanism of the intraocular surgery instrument holder 1 will be described. Note that in the following description, Fig. 1 will also appropriately be referred to.

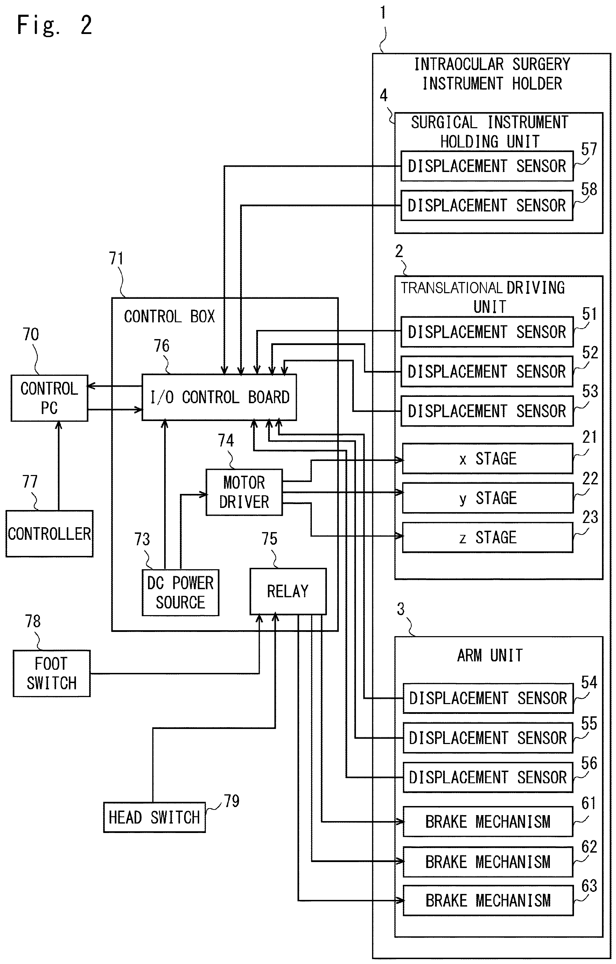

Fig. 2 is a block diagram illustrating the control mechanism of the intraocular surgery instrument holder 1. As illustrated in Fig. 2, a control PC (personal computer) 70 is connected with the intraocular surgery instrument holder 1 via a control box 71. The control box 1 is connected with the control PC 70 by a LAN cable. With the control PC 70, a controller 77 is connected by which the surgeon performs an operation for movement of the field of view of the endoscope 10 as the intraocular surgery instrument. As the controller 77, for example, a joystick may be used by which an input of direction by a lever may be performed.

The control box 71 houses a DC power source 73, a motor driver 74, a relay 75, an I/O (input/output) control board 76, and so forth, which are necessary for driving of the translational driving unit 2. The motor driver 74 is connected with each of the motors of the x stage 21, y stage 22, and z stage 23 in the translational driving unit 2.

The relay 75 is connected with the brake mechanisms 61, 62, and 63 inhibiting motions of the joints (the rotational joints 31 and 32 and the linear motion joint 33) in the arm unit 3. Further, with the relay 75, a foot switch 78 is connected which is for performing a switching operation about whether the surgeon actuates or cancels the brake. The foot switch 78 is a switch which is pushed by a foot and is installed at a foot of the surgeon. Actuation of the brake mechanisms 61, 62, and 63 is canceled when the surgeon pushes the foot switch 78 by his/her foot, and the brake mechanisms 61, 62, and 63 are actuated when the foot is released from an upper portion of the foot switch 78. Furthermore, a head switch 79 is connected with the relay 75. The head switch 79 is installed in a pillow on which the patient places his/her head in the surgery and has a construction in which actuation of the brake mechanisms 61, 62, and 63 is canceled when the patient brings up his/her head. The I/O control board 76 is connected with the displacement sensors 51, 52, and 53 of the translational driving unit 2, the displacement sensors 54, 55, and 56 of the arm unit 3, and the displacement sensors 57 and 58 of the surgical instrument holding unit 4.

Next, a description will be made about switching of action modes in the intraocular surgery instrument holder 1 according to this embodiment. Note that in the following description, Fig. 1 will appropriately be referred to for the configuration of the intraocular surgery instrument holder 1, and Fig. 2 will appropriately be referred to the control mechanism of the intraocular surgery instrument holder 1.

The intraocular surgery instrument holder 1 according to this embodiment includes two modes of a coarse movement mode and a fine movement mode. In a case where the endoscope is positioned or the field of view has to be largely changed, the coarse movement mode is selected in which the endoscope is manually moved. On the other hand, in a case where the field of view of the endoscope is finely moved in giving a treatment, the fine movement mode is selected in which the endoscope is automatically moved. Switching between the coarse movement mode and the fine movement mode is performed by an operation of the foot switch 78 by the surgeon.

When the surgeon does not push the foot switch 78 by his/her foot, the brake mechanisms 61, 62, and 63 are in actuated states (a state of the fine movement mode). In this state, because motions of the joints (the rotational joints 31 and 32 and the linear motion joint 33) in the arm unit 3 are inhibited, the surgeon may not manually move the position of the endoscope 10 as the intraocular surgery instrument. Thus, movement of the position of the endoscope 10 as the intraocular surgery instrument is performed by driving of the translational driving unit 2. When the translational driving unit 2 is driven, the brake mechanisms 61, 62, and 63 may be actuated inter-connectedly. Accordingly, movement of the arm unit 3 may more certainly be inhibited in fine movement.

Note that the brake mechanisms 61, 62, and 63 are actuated not only in a case where the joints (the rotational joints 31 and 32 and the linear motion joint 33) in the arm unit 3 are completely locked. Like so-called "half clutch" in an automobile, the brake mechanisms 61, 62, and 63 may be configured such that the extent that movement of the arm unit 3 is inhibited may be adjusted. In such a configuration, for example, it becomes possible to actuate the brake mechanisms 61, 62, and 63 such that a moderate resistance is given to the hand of the surgeon when the surgeon is manually moving the arm unit 3 in the coarse movement mode, and this facilitates positioning of the endoscope 10 as the intraocular surgery instrument.

As described above, the movable range of the translational driving unit 2 is to an extent that is necessary and sufficient for observation of the interior of the eyeball 90 by the endoscope 10, for example. By setting the movable range of the translational driving unit 2 as described above, if by any case malfunction occurs in an operation in the fine movement mode, the eyeball 90 is not damaged due to large movement of the endoscope 10 as the intraocular surgery instrument.

On the other hand, when the surgeon pushes the foot switch 78 by his/her foot, all of the brake mechanisms 61, 62, and 63 become a state where actuation of brakes is canceled (a state of the coarse movement mode). In this state, because motions of the joints (the rotational joints 31 and 32 and the linear motion joint 33) in the arm unit 3 are not inhibited, the surgeon may manually move the position of the endoscope 10 as the intraocular surgery instrument.

Because a tip part of the endoscope 10 is inserted into a hole formed in the eyeball 90 of the patient in positioning of the endoscope 10 as the intraocular surgery instrument, in movement in positioning of the endoscope 10, the position of the endoscope 10 has to be largely moved compared to a case of movement of the field of view of the endoscope 10 in giving a treatment. Thus, as described above, the movable range of the arm unit 3 is set wider than the movable range of the translational driving unit 2.

The movable range of the arm unit 3 is to an extent that the endoscope 10 as the intraocular surgery instrument may be inserted into the hole formed in the eyeball 90 and the endoscope 10 may be retracted when a microscope is used in giving a treatment. For example, the movable range of the linear motion joint 33 in the arm unit 3 is set to approximately 150 mm. For example, when it is assumed that the distance from the fixing mechanism 24 fixing the intraocular surgery instrument holder 1 to the side periphery of the surgical table 11 to the eyeball 90 of the patient is approximately 250 mm, the length of the arm unit 3 is set to a minimum of approximately 300 mm and a maximum of approximately 450 mm.

Here, a description will be made about a flow of control of the intraocular surgery instrument holder 1 for moving the field of view of the endoscope 10 as the intraocular surgery instrument in the fine movement mode. Note that in the following description, Fig. 1 will appropriately be referred to for the configuration of the intraocular surgery instrument holder 1, and Fig. 2 will appropriately be referred to the control mechanism of the intraocular surgery instrument holder 1.

First, the surgeon gives an operation instruction to the control PC 70 via the controller 77. Next, the control PC 70 receiving the operation instruction reads information from each of the displacement sensors (the displacement amount of each of the joints), the information being input to the I/O control board 76.

The control PC 70 runs a control program and calculates the present position of the endoscope 10 based on the displacement amounts of the joints that are respectively detected by the displacement sensors. Note that the present position of the endoscope 10 is calculated by direct kinematics. Then, the position of the endoscope 10 that has already been moved is calculated based on the calculated present position of the endoscope 10 and the operation instruction, and an action amount of each of the motors of the x stage 21, y stage 22, and z stage 23 is calculated.

The control PC 70 transmits the calculated action amount of each of the motors of the x stage 21, y stage 22, and z stage 23 to the I/O control board 76. Then, the I/O control board 76 causes each of the motors of the x stage 21, y stage 22, and z stage 23 to act by only the calculated action amount.

Subsequently, a series of flow is repeated in which the surgeon gives the operation instruction to the control PC 70 via the controller 77 and the I/O control board 76 causes the x stage 21, y stage 22, and z stage 23 to act by only the action amounts corresponding to the operation instruction. Note that a control period is 1 kHz, for example.

Next, a description will be made about movement of the field of view of the endoscope 10 as the intraocular surgery instrument in the fine movement mode.

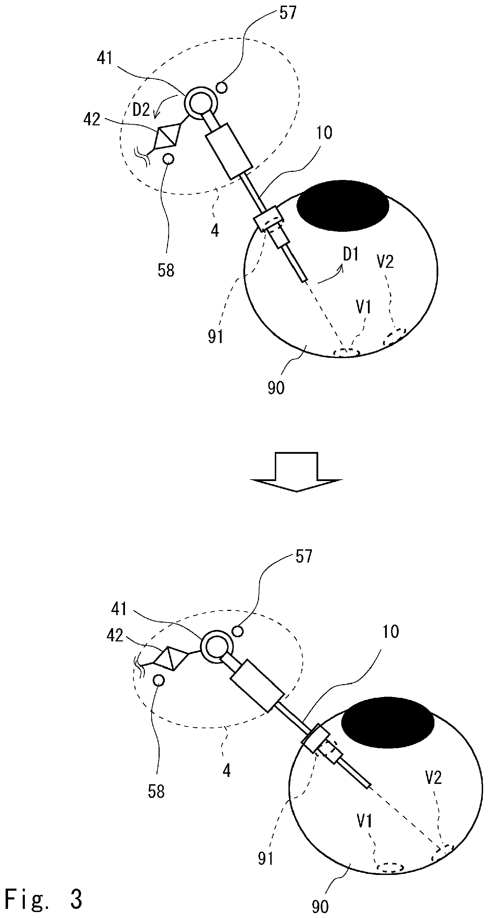

Fig. 3 is a schematic diagram explaining movement of the field of view of the endoscope 10 in up, down, left, and right directions in the fine movement mode. As illustrated in the upper stage of Fig. 3, it is assumed that a region V1 in the interior of the eyeball 90 is observed by the endoscope 10. As described above, the rotational joint 41 in the surgical instrument holding unit 4 is the passive motion joint and may thus indirectly be moved by driving of the translational driving unit 2. When the position of the rotational joint 41 in the surgical instrument holding unit 4 is moved in the direction of an arrow D2, the endoscope 10 rotates around the opening part 91 as the center, and a tip end of the endoscope 10 moves in the direction of an arrow D1. Accordingly, as illustrated in the lower stage of Fig. 3, an observation region of the endoscope 10 is changed to a region V2. As described above, the endoscope 10 is caused to perform a turning motion around the opening part 91 as the center by driving of the translational driving unit 2, and the field of view of the endoscope 10 may thereby be moved up, down, left, and right.

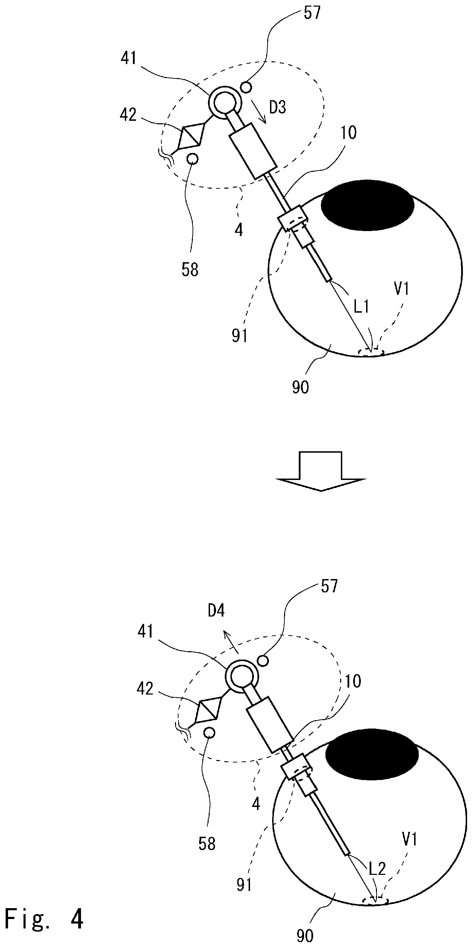

Fig. 4 is a schematic diagram explaining enlargement and reduction (zoom-in and zoom-out) of the field of view of the endoscope 10 in the fine movement mode. As illustrated in the upper stage of Fig. 4, it is assumed that the distance from the tip end of the endoscope 10 to the region V1 in the interior of the eyeball 90 is L1. From this state, when the position of the rotational joint 41 in the surgical instrument holding unit 4 is moved in the direction of an arrow D3, as illustrated in the lower stage of Fig. 4, the distance from the tip end of the endoscope 10 to the region V1 in the interior of the eyeball 90 approaches L2 (L2 < L1). Accordingly, the field of view of the endoscope 10 may be enlarged. From this state, when the position of the rotational joint 41 in the surgical instrument holding unit 4 is moved in the direction of an arrow D4, the field of view of the endoscope 10 may be reduced.

In the foregoing, the intraocular surgery instrument holder 1 is divided such that the translational driving unit 2 serves for movement of the field of view (fine movement) of the endoscope 10 as the intraocular surgery instrument in giving a treatment and the arm unit 3 serves for movement (coarse movement) of the endoscope 10 when the endoscope is positioned or the field of view has to be largely changed. As described above, a movement mechanism for fine movement (here, the translational driving unit 2) and a movement mechanism for coarse movement (here, the arm unit 3) are divided from each other, and physical movable ranges may thereby separately be set for fine movement and coarse movement. By setting the movable range of the translational driving unit 2 short compared to the movable range of the arm unit 3, even if the translational driving unit 2 malfunctions when the translational driving unit 2 causes the endoscope 10 to automatically perform fine movement, risks of damaging the eyeball 90 due to large movement of the endoscope 10 as the intraocular surgery instrument may be lowered as much as possible. Further, movement for a long distance may quickly be performed in coarse movement. Furthermore, because the arm unit 3 is provided with the brake mechanisms 61, 62, and 63, movement of the arm unit 3 may be restricted in a case where the arm unit 3 is not desired to be moved such as a case where the translational driving unit 2 causes the endoscope 10 as the intraocular surgery instrument to perform fine movement.

Note that the present invention is not limited to the above embodiment but may appropriately be modified without departing from the scope of the invention as defined by the claims. In the above embodiment, a description is made about a case where the intraocular surgery instrument is the endoscope; however, the intraocular surgery instrument is not limited to this. For example, the intraocular surgery instrument may be replaced by forceps, a light, an aspirator, or the like.

Further, in the present invention, the translational driving unit is a driving mechanism configured to be capable of movement with multiple degrees of freedom, and the arm unit is a passive motion mechanism configured to be capable of movement with multiple degrees of freedom. In the above embodiment, a description is made on the assumption that the translational driving unit is a driving mechanism configured to be capable of movement with three degrees of translational freedom and the arm unit is configured to be capable of movement with two degrees of rotational freedom and one degree of translational freedom in a polar coordinate system; however, configurations are not limited to those. Multiple degrees of freedom mentioned here means at least three degrees of freedom and may be three degrees of rotational freedom, three degrees of translational freedom, and so forth, for example.

The present application claims priority based on Japanese Patent Application No. 2018-088603, filed on May 2, 2018.

### Reference Signs List

1 intraocular surgery instrument holder
2 translational driving unit
3 arm unit
4 surgical instrument holding unit
10 endoscope
11 surgical table
21 x stage
22 y stage
23 z stage
24 fixing mechanism
31, 32, 41, 42 rotational joint
33 linear motion joint
51, 52, 53, 54, 56, 57, 58 displacement sensor
60 self-weight compensation mechanism
61, 62, 63 brake mechanism
70 control PC
71 control box
73 DC power source
74 motor driver
75 relay
76 I/O control board
77 controller
78 foot switch
79 head switch

## Claims

1. An intraocular surgery instrument holder (1) comprising
a translational driving unit (2) that is a driving mechanism configured to be capable of movement with multiple degrees of freedom;
an arm unit (3) that is a passive motion mechanism coupled with the translational driving unit and is configured to be capable of movement with multiple degrees of freedom; and
a surgical instrument holding unit (4) that is a passive gimbal mechanism coupled with one end of the arm unit, and is configured to hold an intraocular surgery instrument (10) and to be capable of movement with two degrees of rotational freedom with an opening part of a hole being a fixed point, the hole being formed in an eyeball for insertion of the intraocular surgery instrument, wherein
a brake mechanism (61, 62, 63) for inhibiting movement of the arm unit is provided to the arm unit,
wherein when the translational driving unit (2) is driven, the brake mechanism (61, 62, 63) is actuated inter-connectedly.

2. The intraocular surgery instrument holder according to Claim 1, wherein the multiple degrees of freedom are at least three degrees of freedom.

3. The intraocular surgery instrument holder according to any one of Claims 1 to 2, wherein
a self-weight compensation mechanism (60) for supporting self-weights of the arm unit, the surgical instrument holding unit, and the intraocular surgery instrument is provided to the arm unit, and
a force of the self-weight compensation mechanism is exerted to lift a tip end of the arm unit perpendicularly upward with respect to the arm unit in a state where the brake mechanism is not actuated.

## Patentansprüche

1. Halterung für ein intraokulares chirurgisches Instrument (1), umfassend:
eine translatorische Antriebseinheit (2), die ein Antriebsmechanismus ist, der so konfiguriert ist, dass er zu einer Bewegung mit mehreren Freiheitsgraden fähig ist;
eine Armeinheit (3), bei der es sich um einen passiven Bewegungsmechanismus handelt, der mit der translatorischen Antriebseinheit gekoppelt und so konfiguriert ist, dass er sich mit mehreren Freiheitsgraden bewegen kann; und
eine Halteeinheit (4) für ein chirurgisches Instrument, bei der es sich um einen passiven kardanischen Mechanismus handelt, der mit einem Ende der Armeinheit gekoppelt ist, und die so konfiguriert ist, dass sie ein intraokulares chirurgisches Instrument (10) hält und in der Lage ist, sich in zwei Rotationsfreiheitsgraden in einem Öffnungsteil eines Lochs zu bewegen, das ein feststehender Punkt ist, wobei das Loch in einem Augapfel zum Einführen des intraokularen chirurgischen Instruments ausgebildet ist, wobei
ein Bremsmechanismus (61, 62, 63) zum Sperren der Bewegung der Armeinheit an der Armeinheit vorgesehen ist,
wobei, wenn die translatorische Antriebseinheit (2) angetrieben wird, der Bremsmechanismus (61, 62, 63) ineinandergreifend betätigt wird.

2. Halterung für intraokulares chirurgisches Instrument nach Anspruch 1, wobei die mehreren Freiheitsgrade mindestens drei Freiheitsgrade sind.

3. Halterung für intraokulares chirurgisches Instrument nach einem der Ansprüche 1 bis 2, wobei
ein Mechanismus (60) zum Ausgleich des Eigengewichts zur Unterstützung der Eigengewichte der Armeinheit, der Halteeinheit für das chirurgische Instrument und des intraokularen chirurgischen Instruments an der Armeinheit, und
wobei eine Kraft des Mechanismus zum Ausgleich des Eigengewichts ausgeübt wird, um ein Spitzenende der Armeinheit senkrecht nach oben in Bezug auf die Armeinheit in einem Zustand anzuheben, in dem der Bremsmechanismus nicht betätigt ist.

## Revendications

1. Un support (1) d'instrument de chirurgie intraoculaire comprenant :
une unité d'entraînement en translation (2) qui est un mécanisme d'entraînement configuré pour être capable de se déplacer avec plusieurs degrés de liberté ;
une unité de bras (3) qui est un mécanisme de mouvement passif couplé à l'unité d'entraînement en translation et configuré pour être capable de se déplacer à plusieurs degrés de liberté ; et
une unité de maintien d'instrument chirurgical (4) qui est un mécanisme passif à cardan couplé à une extrémité de l'unité de bras, et qui est configurée pour tenir un instrument de chirurgie intraoculaire (10), et pour être capable de se déplacer avec deux degrés de liberté de rotation avec une partie d'ouverture d'un trou étant un point fixe, le trou étant formé dans un globe oculaire pour l'insertion de l'instrument de chirurgie intraoculaire, dans lequel
un mécanisme de freinage (61, 62, 63) pour inhiber le mouvement de l'unité de bras est fourni à l'unité de bras,
Lorsque l'unité d'entraînement en translation (2) est entraînée, le mécanisme de freinage (61, 62, 63) est actionné de manière interconnectée.

2. Le support d'instrument de chirurgie intraoculaire selon la revendication 1, dans lequel les multiples degrés de liberté sont au moins trois degrés de liberté.

3. Le support d'instrument de chirurgie intraoculaire selon l'une des revendications 1 à 2, dans lequel
un mécanisme de compensation du poids propre (60) pour supporter le poids propre de l'unité de bras, de l'unité de maintien de l'instrument chirurgical et de l'instrument de chirurgie intraoculaire à l'unité de bras, et
une force du mécanisme de compensation du poids propre est exercée pour soulever une extrémité de l'unité du bras perpendiculairement vers le haut par rapport à l'unité du bras dans un état où le mécanisme de freinage n'est pas actionné.
